# EUROPEAN PATENT SPECIFICATION

(11) **EP 1 792 966 B1**
(45) Date of publication and mention of the grant of the patent: **28.06.2017**
(21) Application number: 06255793.9
(22) Date of filing: 13.11.2006
(51) Int. Cl.: C09K 15/00, A61L 31/16, A61L 31/14, A61L 29/14, A61L 29/16, C08F 220/18, C08F 220/26, C08F 212/14, C08F 224/00, C08F 12/32, C07C 39/21, C08F 8/14, C08F 8/12

(54) **MEDICAL DEVICE COMPRISING RESVERATROL FUNCTIONALIZED POLYMERIC COATING**
MEDIZINISCHE VORRICHTUNG ENTHALTEND EINE RESEVERATROL-FUNKTIONALISIERTE POLYMERBESCHICHTUNG
DISPOSITIF MÉDICAL COMPRENANT UN REVÊTEMENT POLYMÈRE FONCTIONNALISÉ AVEC RESVÉRATROL

(30) Priority: 01.12.2005 US 291791
(43) Date of publication of application: 06.06.2007
(73) Proprietor: Cordis Corporation, Miami Lakes, FL 33014 (US)
(72) Inventor: Zhao, Jonathon Z., Belle Mead, NJ 08502 (US)
(74) Representative: Prock, Thomas

(56) References cited:
- EP-A- 1 338 278
- EP-A- 1 586 338
- WO-A-01/05781
- WO-A-2007/002318
- WO-A2-2004/050795
- US-A- 5 185 407
- US-A- 5 258 422
- US-A1- 2004 043 323
- US-A1- 2004 199 241
- KUCZKOWSKI J A ET AL: "POLYMER-BOUND ANTIOXIDANT" RUBBER CHEMISTRY AND TECHNOLOGY, ACS, AKRON, OH, US, vol. 57, no. 3, July 1984 (1984-07), pages 621-651, XP009029436 ISSN: 0035-9475
- ATKINSON D ET AL: "A MODEL POLYMER-BOUND ANTIOXIDANT SYSTEM FOR LUBRICANTS" EUROPEAN POLYMER JOURNAL, PERGAMON PRESS LTD. OXFORD, GB, vol. 28, no. 12, 1992, pages 1569-1575, XP007900079 ISSN: 0014-3057
- AUER MARKKU ET AL: "Synthesis of novel-dl-alpha -tocopherol-based and sterically-hindered-phenol-based monomers and their utilization in copolymerizations over metallocene/MAO catalyst systems. A strategy to remove concerns about additive compatibility and migration" MACROMOLECULES; MACROMOLECULES NOV 4 2003, vol. 36, no. 22, 4 November 2003 (2003-11-04), pages 8346-8352, XP002439884
- SHIMODA, FUMIO: "An ENDOR study of the hindered internal rotation of the cyclopropyl group in the 2,6-di-tert-butyl-4-cyclopropylphenoxy radical" BULLETIN OF THE CHEMICAL SOCIETY OF JAPAN , 53(7), 1853-9 CODEN: BCSJA8; ISSN: 0009-2673, 1980, XP002439885
- LEE HYUN JUNG ET AL: "Syntheses and radical scavenging activities of resveratrol derivatives." BIOORGANIC & MEDICINAL CHEMISTRY LETTERS 19 JAN 2004, vol. 14, no. 2, 19 January 2004 (2004-01-19), pages 463-466, XP002439886 ISSN: 0960-894X
- TAKAYA Y ET AL: "Biomimic transformation of resveratrol" TETRAHEDRON 24 OCT 2005 UNITED KINGDOM, vol. 61, no. 43, 24 October 2005 (2005-10-24), pages 10285-10290, XP005087892 ISSN: 0040-4020
- ZHANG ZHAOJUN ET AL: "Synthesis of mono- and di-O-beta -D-glucopyranoside conjugates of (E)-resveratrol" SYNTHESIS; SYNTHESIS APR 19 2006, no. 8, 19 April 2006 (2006-04-19), pages 1301-1306, XP002439887

## Description

Polymeric compositions that can be used for controlled release or delivery of one or more pharmacologically active agents are disclosed herein. The present invention relates to implantable medical devices that are either formed of or coated with one or more such polymeric compositions. Such implantable medical devices can be used for local administration and controlled release of various pharmacologically active agents that are effective in treating or preventing disease, promoting healing and endothelialization, and/or minimizing or substantially eliminating a biological organism's immune reaction to the introduction of such medical devices. More specifically, such medical devices are useful for local administration and controlled release of drug/drug combinations for the prevention and treatment of vascular disease, especially vascular disease caused by injury.

In recent years, implantable medical devices that contain one or more pharmacologically active agents for local administration and controlled release of such pharmacologically active agents have gained more and more acceptance in the medical device industry. Typically, biocompatible homopolymers or copolymers, either non-degradable or degradable, are used for forming or coating at least a part of the medical devices, so as to provide a biocompatible polymeric matrix for controlled release of one or more pharmacologically active agents from the device surface.

However, certain pharmacologically active agents, such as rapamycin, paclitaxel, trans-retinoic acid, etc., contain one or more unsaturated bonds that are vulnerable to oxidative degradation. Consequentially, such pharmacologically active agents may gradually lose their potency and biological activities over time, resulting in significantly shortened shelf life.

There is therefore an urgent need to stabilize the oxidation-susceptible pharmacologically active agents contained in the biocompatible polymeric matrix, in order to extend the shelf life of such pharmacologically active agents.

The present invention relates to implantable medical devices that are either formed of or coated with one or more polymeric compositions that comprise a biocompatible polymeric matrix that contains at least one pharmacologically active agent that is susceptible to oxidative degradation, wherein the biocompatible polymeric matrix comprises at least a first polymeric material with at least one active antioxidant functional moiety covalently bound thereto in sufficient amount for protecting the at least one pharmacologically active agent against oxidative degradation. The antioxidant functional moiety includes trans-resveratrol or cis-resveratrol.

Optionally, the biocompatible polymeric matrix further comprises a second polymeric material that is essentially free of covalently bound active antioxidant functional moiety.

The term "polymer" or "polymeric" as used herein refers to any material, composition, structure, or article that comprises one or more polymers, which can be homopolymers, copolymers, or polymer blends.

The term "biocompatible" as used herein refers to any material, composition, structure, or article that have essentially no toxic or injurious impact on the living tissues or living systems which the material, composition, structure, or article is in contact with and produce essentially no immunological response in such living tissues or living systems. More particularly, the material, composition, structure, or article has essentially no adverse impact on the growth and any other desired characteristics of the cells of the living tissues or living systems that are in contact with the material, composition, structure, or article. Generally, the methods for testing the biocompatibility of a material, composition, structure, or article is well known in the art.

The phrase "active antioxidant functional moiety" as used herein refers to a functional moiety that actively exhibits antioxidant functionality, in distinction with a functional moiety having only dormant or latent antioxidant functionality that needs to be activated. For example, a phenol or substituted phenol moiety that contains a free hydroxyl functional group actively exhibits antioxidant functionality and is therefore considered an active antioxidant functional moiety within the meaning of the present invention. In contrast, a protected phenol moiety having its hydroxyl functional group being protected or substituted by another functional group exhibits no antioxidant functionality, until it is activated by a process that forms a free hydroxyl functional group therein.

The pharmacologically active agents that can be incorporated into the biocompatible polymeric matrix and be protected by the covalently bound active antioxidant functional moiety include, for example, trans-retinoic acid, paclitaxel, paclitaxel derivatives, rapamycin, and rapamycin derivatives that comprise at least one domain that binds to a mammalian target of rapamycin (mTOR).

The composition can include a covalently bound active antioxidant functional moiety is selected from the group consisting of phenolics, vitamin E, vitamin C, vitamin A, vitamin K, and derivatives thereof. More preferably, the covalently bound active antioxidant functional moiety comprises at least one of phenol, butylated hydroxytoluene (BHT), probucol, catecol, resorcinol, hydroquinon, mycophenolic acid (MPA), tyrosine, and derivatives thereof. The term "derivatives" as used herein broadly covers any derivative compounds of a specific compound having a desired property, which can be formed by addition, substitution, and/or deletion of one or more functional groups, wherein such derivative compounds exhibit the same or similar property as the specific compound.

The first polymeric material may comprise any number of covalently bound active antioxidant functional moieties, either of the same type or different types. In a preferred but not necessary embodiment of the present invention, there are two or more different types of active antioxidant functional moieties covalently bound to the first polymeric material.

Further, the active antioxidant functional moiety of the present invention may be covalently bound to the first polymeric material through a side-chain linking group. Such a side-chain linking group can have any formula or structure, as long as it provides sufficient spatial separation between the active antioxidant functional moiety and the polymeric backbone of the first polymeric material to avoid steric hindrance, without adversely impact the functionality of the active antioxidant functional moiety. Preferably, but not necessarily, the side-chain linking group is selected from the group consisting of straight or branched C₁-C₂₀ alkyls, C₁-C₂₀ alkylenes, C₁-C₂₀ oxaalkylenes, C₁-C₂₀ oligo-oxaalkylenes, C₁-C₂₀ alkyleneoxaalkylenes, and C₁-C₂₀ alkylene oligo-oxaalkylenes, optionally with one or more fluorine substitution groups.

The first polymeric material that forms at least a portion of the biocompatible polymeric matrix of the present invention may comprise any biocompatible polymers, including, but not limited to biocompatible addition polymers and biocompatible condensation polymers. Preferably, the first polymeric material comprises a biostable or bioabsorbable polymer. Biostable polymers that are suitable for use in this invention include, but are not limited to: polyurethane, silicones, polyesters, polyolefins, polyamides, poly(esteramide), polycaprolactam, polyimide, polyvinyl chloride, polyvinyl methyl ether, polyvinyl alcohol, acrylic polymers and copolymers, polyacrylonitrile; polystyrene copolymers of vinyl monomers with olefins (such as styrene acrylonitrile copolymers, ethylene methyl methacrylate copolymers, ethylene vinyl acetate), polyethers, rayons, cellulosics (such as cellulose acetate, cellulose nitrate, cellulose propionate, etc.), parylene and derivatives thereof; and mixtures and copolymers of the foregoing. Bioabsorbable polymers that can be used in this invention include, but are not limited to: poly(L-lactic acid), poly(DL-lactic acid), polycaprolactone, poly(hydroxy butyrate), polyglycolide, poly(diaxanone), poly(hydroxy valerate), polyorthoester; copolymers such as poly (lactide-co-glycolide), poly(hydroxy butyrate-co-valerate), poly(glycolide-co-trimethylene carbonate); polyanhydrides; polyphosphoester; poly(phosphoester-urethane); poly(amino acids); polycyanoacrylates; biomolecules such as fibrin, fibrinogen, cellulose, starch, collagen and hyaluronic acid; and mixtures and copolymers of the foregoing.

More preferably, the first polymeric material is selected from the group consisting of biocompatible polyvinyl polymer, polyester, polyamide, poly(esteramide), polyurethane, poly(butyl methacrylate), poly(2-hydroxyethylmethacrylate), or combinations thereof. More preferably, the first polymeric material is a biocompatible vinyl.

In a particularly preferred embodiment of the present invention, the first polymeric material is a poly(butyl methacrylate) and poly(2-hydroxyethylmethacrylate) copolymer having said at least one active antioxidant functional moiety covalently bounded thereto. For example, such a copolymer may have at least one section with the following formula: 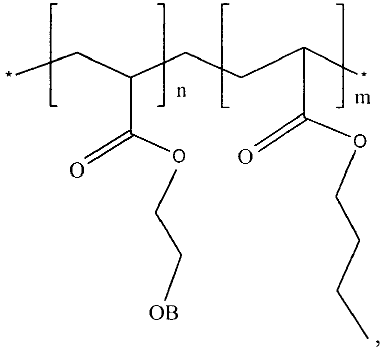 wherein B is an active antioxidant functional moiety, and wherein both n and m ≥ 1.

The biocompatible polymeric matrix of the present invention preferably, but not necessarily, comprises the second polymeric material as mention hereinabove. The second polymeric material may comprise any biocompatible polymers, including, but not limited to biocompatible addition polymers and biocompatible condensation polymers. Preferably, the second polymeric material is a biostable or bioabsorbable polymer, as described hereinabove. More preferably, the second polymeric material is selected from the group consisting of biocompatible polyvinyl, polyester, polyamide, poly(esteramide), polyurethane, poly(butyl methacrylate), poly(2-hydroxyethylmethacrylate), or combinations thereof. More preferably, the second polymeric material is a biocompatible vinyl. The second polymeric material may have substantially the same chemical structure as the first polymeric material, with the exception that the second polymeric material is essentially free of any covalently bound active antioxidant functional moiety.

The present invention relates to a medical device that comprises an
implantable structure, while at least a portion of the implantable structure either comprises or is coated by the polymeric composition as described hereinabove. The implantable structure is preferably selected from the group consisting of stents, stent grafts, anastomosis devices, vascular grafts, vascular patches, AV shunts, catheters, guide wires, balloons, and filters.

Also disclosed herein is a method for forming a medical device, comprising:
providing a polymeric composition comprising a biocompatible polymeric matrix that contains at least one pharmacologically active agent that is susceptible to oxidative degradation, wherein the biocompatible polymeric matrix comprises at least a first polymeric material with an active antioxidant functional moiety covalently bound thereto in sufficient amount for protecting the at least one pharmacologically active agent against oxidative degradation, and optionally, the biocompatible polymeric matrix further comprising a second polymeric material that is essentially free of covalently bound active antioxidant functional moiety; and
using the polymeric composition to form or coat at least a portion of an implantable structure.

Other aspects, features and advantages of the invention will be more fully apparent from the ensuing disclosure and appended claims.

In the following description, numerous specific details are set forth, such as particular materials, compositions, formula, structures, devices, and methods for fabricating or using same, in order to provide a thorough understanding of the present invention. However, it will be appreciated by one of ordinary skill in the art that the invention may be practiced without these specific details. In other instances, well-known materials, structures or processing steps have not been described in detail in order to avoid obscuring the invention.

As mentioned hereinabove, certain pharmacologically active agents contained in implantable medical devices are vulnerable to oxidative degradation, and they may gradually lose their potency over time due to oxidation of free radicals contained therein. It is therefore important to stabilize such free radicals in order to prevent degradation of the pharmacologically active agents.

One possible approach is to use a polymeric composition that contains both the oxidation-sensitive pharmacologically active agents and one or more antioxidant additives (e.g., butylated hydroxytoluene, butylated hydroxyanisole, tocopherol, and probucol, etc.) for forming or coating at least a part of the implantable medical devices. The antioxidant additives terminates the free radicals contained in the pharmacologically active agents by donating electrons or hydrogen to the free radicals, thereby stabilizing the pharmacologically active agents and protecting the pharmacologically active agents against potential oxidative attack.

However, such an approach requires highly optimized processing conditions, including, but not limited to: temperatures, pressures, solvent systems, agitation rates, etc., for achieving a sufficiently homogeneous mixture of the biocompatible polymer, the pharmacologically active agent(s), and the antioxidant additive(s). Sub-optimal processing conditions may lead to deleterious phase separation between the mixed components due to the differences in their respective physical and chemical properties. Further, the antioxidant additive(s) have relatively low molecular weights in comparison with the pharmacologically active agent(s) and the biocompatible polymer. Correspondingly, such antioxidant additive(s) have relatively low melting or sublimation points and may gradually leach out of or evaporate from the polymeric matrix overtime, thereby losing the capability of protecting the pharmacologically active agents.

The present invention therefore provides a polymeric composition, which contains a biocompatible polymeric matrix that contains a first polymeric material with at least one active antioxidant functional moiety covalently bonded thereto, for supporting the oxidation-susceptible pharmacologically active agents as well as protecting such pharmacologically active agents against oxidative degradation. The covalent bonding between the active antioxidant functional moiety and the first polymeric material effectively prevents phase separation and significantly reduces leaching or evaporation of the antioxidant from the polymeric matrix. Consequentially, the active antioxidant functional moiety is better integrated into the polymeric composition of the present invention to provide long-lasting protection for the oxidation-sensitive pharmacologically active agents.

The first polymer contained by the biocompatible polymeric matrix of the present invention may comprise at least a section having the following generic formula: 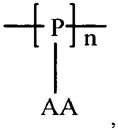 or 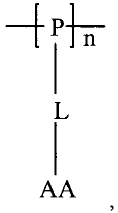 while P represents repeating units that form at least a section of the polymeric backbone of the first polymer, n is an integer ≥1, AA represents active antioxidant functional moieties, and L represents linking groups that links the active antioxidant functional moieties to the polymeric backbone via covalent bonds.

The first polymer as described hereinabove can be any suitable polymer, including, but not limited to, addition polymer and condensation polymer. It may be selected from the group consisting of: (1) homopolymers that contain substantially the same repeating units and substantially the same covalently bonded active antioxidant functional moieties, (2) homopolymers that contain substantially the same repeating units but two or more different covalently bonded active antioxidant functional moieties, (3) copolymers that contain two or more different repeating units but substantially the same covalently bonded active antioxidant functional moieties, or (4) copolymers that contain two or more different repeating units and two or more different covalently bonded active antioxidant functional moieties, which can be represented by the general formula of: 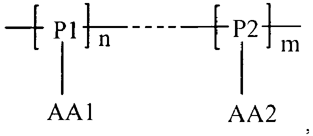 or 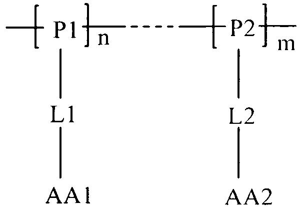 wherein n and m are both integers ≥1, P1 and P2 represent either the same or different repeating units that form the polymeric backbone, AA1 and AA2 represent either the same or different active antioxidant functional moieties, and L1 and L2 are either the same or different linking groups.

The above-described polymers that contain covalently bonded antioxidant functional moieties can be used for forming a polymeric matrix that both supports as well as stabilizes those pharmacologically active agents that are oxidation-sensitive, including, but not limited to: trans-retinoic acid, paclitaxel, paclitaxel derivatives, rapamycin, rapamycin derivatives that comprise at least one domain that binds to a mammalian target of rapamycin (mTOR), or any other pharmacologically active agents that contain one or more unsaturated bonds that are vulnerable to oxidation attack.

Preferably, the pharmacologically active agents used for practicing the present invention comprise at least rapamycin. Rapamycin, also referred to as sirolimus, is a macrocyclic triene antibiotic produced by Streptomyces hygroscopicus as disclosed in US-3,929,992. It has been found that rapamycin, among other things, inhibits the proliferation of vascular smooth muscle cells *in vivo.* Accordingly, rapamycin may be utilized in treating intimal smooth muscle cell hyperplasia, restenosis, and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls. Rapamycin reduces vascular hyperplasia by antagonizing smooth muscle proliferation in response to mitogenic signals that are released during an angioplasty-induced injury. Inhibition of growth factor and cytokine mediated smooth muscle proliferation at the late G1 phase of the cell cycle is believed to be the domain mechanism of action of rapamycin. However, rapamycin is also known to prevent T-cell proliferation and differentiation when administered systematically, and it therefore can be used as an immunosuppressant for preventing graft rejection.

Rapamycin has the following chemical structure:

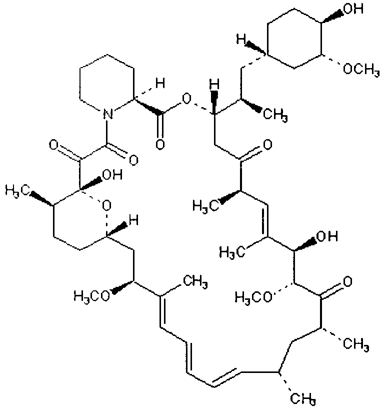

Specifically, a functional domain of the rapamycin molecule, which includes the three double bonds, is capable of binding to the mammalian target of rapamycin (mTOR), a kinase required for cell-cycle progression. Inhibition of the mTOR kinase activity by rapamycin blocks T-cell activation and proinflammatory cytokine secretion and is the underlying mechanism responsible for the immunosuppressant and anti-hyperplasic activities of rapamycin. Therefore, rapamycin derivatives having similar functional domains with the three double bonds, such as rapamycin ester, everolimus, tacrolimus, pimecrolimus, and wortmannin, are also capable of binding to the mTOR kinase and exhibit immunosuppressant and anti-hyperplasic activities. More importantly, since such rapamycin derivatives also contain the unsaturated double bonds that are susceptible to oxidation, they can also be supported and protected by the above-described polymeric composition with covalently bonded active antioxidant functional moieties.

The covalently bound active antioxidant functional moieties used in the present invention protect and stabilize the oxidation-sensitive pharmacologically active agents, by donating electrons or hydrogen to the free radicals contained in such agents to terminate such free radicals. Suitable antioxidant functional moieties include, but are not limited to phenolics, vitamin E, vitamin C, vitamin A, vitamin K, and derivatives thereof.

Phenolics are a class of chemical compounds that contain at least one hydroxyl group attached to an aromatic hydrocarbon group. Various phenolics, such as phenol, butylated hydroxytoluene (BHT), probucol, catecol, resorcinol, hydroquinon, mycophenolic acid (MPA), tyrosine, trans-resveratrol, cis-resveratrol, etc., have well known antioxidant functions and can be readily used for forming the active antioxidant functional moieties of the present invention.

Vitamin E is a fat-soluble vitamin that is also an important antioxidant. Natural vitamin E exists in eight different forms or isomers, including four tocopherols (α-tocopherol, β-tocopherol, γ-tocopherol, and δ-tocopherol) and four tocotrienols (α-tocotrienol, β-tocotrienol, γ-tocotrienol, and δ-tocotrienol). Synthetic vitamin E can be formed by coverting the natural tocopherol isomers into esters using acetic or succinic anhydride and adding methyl groups to yield tocopherol esters such as tocopherol acetate and tocopherol succinate. All vitamin-E isomers comprise a chromanol ring with a hydroxyl group that can donate a free hydrogen atom for reducing free radicals and can be used for forming the active antioxidant functional moieties of the present invention. Further, because the vitamin-E isomers each comprise a long hydrophobic side chain, they can be directly covalently bonded to polymeric molecules to form the polymeric composition of the present invention, without need for any intermediate linking groups.

Other non-phenolic antioxidants that can be used for forming the active antioxidant functional moieties of the present invention include vitamin C (e.g., ascorbic acid, sodium ascorbate or other ascrobates), vitamin A (e.g., retinal, retinal or retinoic acid), vitamin K (including phylloquinone, menaquinone, menadione, and derivatives thereof), etc.

The polymeric composition of the present invention may comprise any number of active antioxidant functional moieties, which can be either the same type or two or more different types. The concentration of active antioxidant functional moieties in such a polymeric composition may vary widely, depending on the specific requirements of the applications. For example, the polymeric composition may comprise from about 0.1% to about 99.9% of active antioxidant functional moieties by total weight of the polymeric composition. However, the mechanical and chemical properties of the polymeric matrix may be comprised by higher concentrations of active antioxidant functional moieties, it is more preferred that the polymeric composition of the present invention comprises from about 0.1 wt% to about 50wt%, more preferably 1 wt% to about 30wt%, and most preferably 5 wt% to about 15wt%, of the active antioxidant functional moieties.

Further, the polymeric composition of the present invention may comprise a combination of any two or more different types of antioxidant functional moieties as described hereinabove, both covalently bound to the first polymeric material. Such combination may provide a broad spectrum of antioxidant effects, and more advantageously, such combination may exhibit synergic antioxidant effects that are stronger than the sum of the individual antioxidants. Also disclosed herein are polymeric compositions comprising combinations of: (1) phenol or substituted phenol and at least one tocopherol; (2) BHT and at least one tocopherol or tocopherol derivative, (3) all naturally exiting vitamin E isomers and/or derivatives thereof; (4) BHT and all naturally existing vitamin E isomers and/or derivatives thereof; (5) all tocotrienols and/or derivatives thereof; (6) at least one vitamin E isomer and vitamin C; (7) BHT and probucol; (9) BHT and MPA; (9) tyrosine and at least one vitamin E isomer.

Also disclosed herein is a first polymeric material contained by the biocompatible polymeric matrix having both a tocopherol-derived antioxidant functional moiety and a phenol functional moiety covalently bound thereto. Specifically, at least a section of the first polymeric material has the following formula: 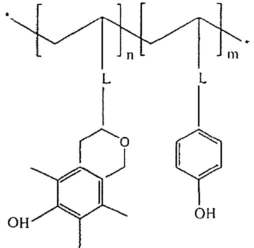 wherein L is an optional linking group, and wherein both n and m are integers >1.

The active antioxidant functional moieties can be covalently bonded to the first polymeric material in either a random manner or a controlled manner, and they may present at any desired section(s) of the polymeric molecules of the first polymeric material, e.g., in the middle or at the terminal ends.

The optional linking group between the active antioxidant functional moiety and the polymeric backbone can be any suitable straight or branched alkyl, alkylene, oxaalkylene, oligo-oxaalkylene, alkyleneoxaalkylene, or alkylene oligo-oxaalkylene that contains from about 1 to about 20 carbon atoms, more preferably with from about 1 to about 10 carbon atoms, and optionally one or more fluorine substitution groups. The linking group functions to reduce steric hindrance between the active antioxidant functional moiety and the polymeric backbone of the first polymeric material, so the length of the linking group can be readily adjusted, depending on the sizes and structures of the active antioxidant functional moiety and the polymeric backbone. Further, the structure of the linking group can be adjusted to optimize the physical and chemical properties of the resulting polymeric molecules, according to specific application requirements.

The first polymeric material of the present invention, to which the active antioxidant functional groups are covalently bonded, may comprise any suitable polymer, copolymer, or polymer blend of sufficient biocompatibility. It can be either an addition polymer or a condensation polymer, and it preferably includes, but is limited to, at least one of polyvinyls, polyesters, polyamides, poly(esteramide), polyurethanes, poly(butyl methacrylate), poly(2-hydroxyethylmethacrylate), and combinations thereof. Preferably, the first polymeric material is a biocompatible homopolymer formed by polymerization of butyl methacrylate (BMA) or 2-hydroxyethyl methacrylate (HEMA) monomers, or any biocompatible copolymer, such as ethylene/vinyl acetate copolymer (EVAc) or BMA/HEMA copolymer.

In one specific embodiment of the present invention, a polymeric precursor compound that contains at least one reactive group is reacted with one or more antioxidants or antioxidant derivates, so as to form the first polymeric material of the present invention with the covalently bonded active antioxidant functional moieties.

For example, a poly(butyl methacrylate/2-hydroxyethyl methacrylate) copolymer precursor compound having the following chemical formula is first provided: 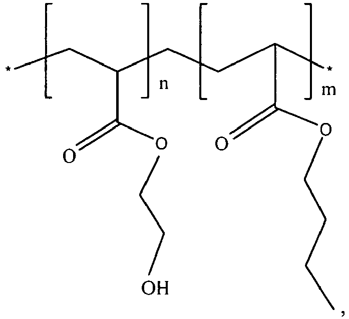 wherein n and m are both integers ≥ 1, and wherein n:m ranges from about 1:9 to 9:1.

Since such a poly(BMA/HEMA) copolymer precursor compound comprises a reactive -OH group, it can be reacted with an active antioxidant to form a poly(BMA/HEMA) copolymer with a covalently bonded active antioxidant functional moiety, as follows: 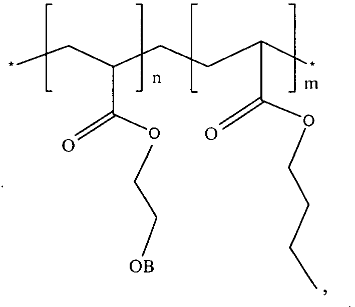 wherein B represents the covalently bonded active antioxidant functional moiety, and n and m both ≥ 1.

In an alternatively embodiment of the present invention, a polymerizable monomer can first be provided, which comprises one or more antioxidant functional moieties already covalently bound to a polymerizable section, either directly or through an intermediate linking group. The polymerizable section can undergo polymerization to form a polymeric backbone of the first polymeric material of the present invention. Because active antioxidant functional moieties typically comprise free electrons or hydrogen that may interfere with the polymerization process, the antioxidant functional moieties contained by the polymerizable monomers of the present invention are temporarily protected, i.e., by one or more capping structures, so as to reduce or minimize their interference with the polymerization process, and the protected antioxidant functional moieties can be readily activated, for example, by a hydrolysis reaction that removes the protective capping structures, to regain the antioxidant functionalities after the polymerization has completed. Such protection and de-protection of the antioxidant functional moieties are known in the polymer industry. See Theodora W. Greene & Peter G.M. Wuts, Protective Groups in Organic Synthesis, 3rd Edition, pp. 246-292 (Wiley 1999).

For example, such a polymerizable monomer may have at least one section having the following general formula: 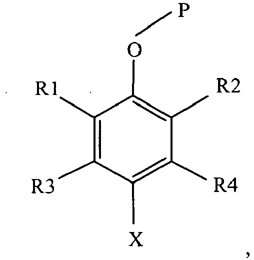 R1, R2, R3, and R4 are independently selected from the group consisting of hydrogen, hydroxyl, C₁-C₈ alkyl, etc. P is a protective functional group (i.e., a capping structure) that can be removed by hydrolysis after polymerization to form a hydroxyl functional group with free hydrogen on the benzyl ring. X is a polymerizable functional group that can undergo polymerization to form a polymeric backbone of the first polymeric material. Such monomers may comprise two or more sections having the above-described formula.

Several exemplary monomers that can be used for forming the first polymeric material of the present invention are specified hereinafter for illustration purposes only, which should not be construed in any manner to limit the broad scope of the present invention.

For example, a 4-acetoxystyrene monomer, which has a chemical formula of: 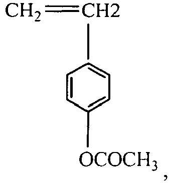 contains a phenol functional moiety that is covalently bonded to a polymerizable vinyl group and is protected by a -COCH₃ cap. Such a monomer can be first polymerized to form a polymeric precursor having a chemical formula of: 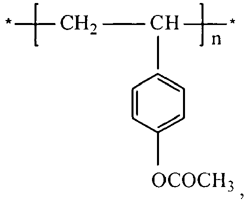 which can be hydrolyzed to form a polymeric molecule with an active phenol functional moiety covalently bonded to a polyvinyl backbone, as follows:

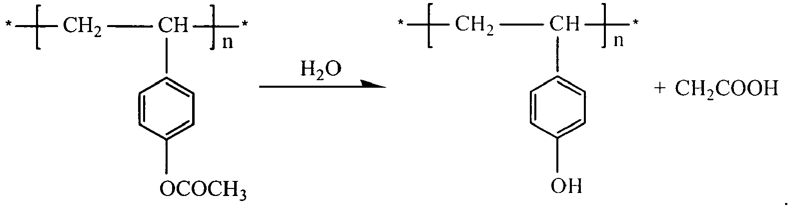

For another example, a 4-acetoxystyrene monomer as described hereinabove can be reacted with a butyl methacrylate (BMA) monomer to form a copolymer as follows: 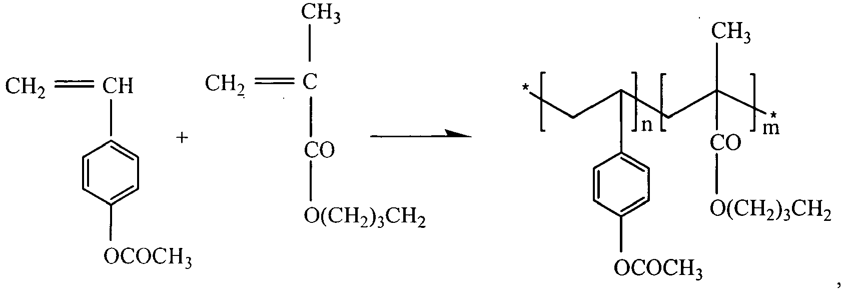 and the copolymer can then be hydrolyzed to activate the phenol functional moiety so as to form a poly(phenol/BMA) copolymer, as follows:

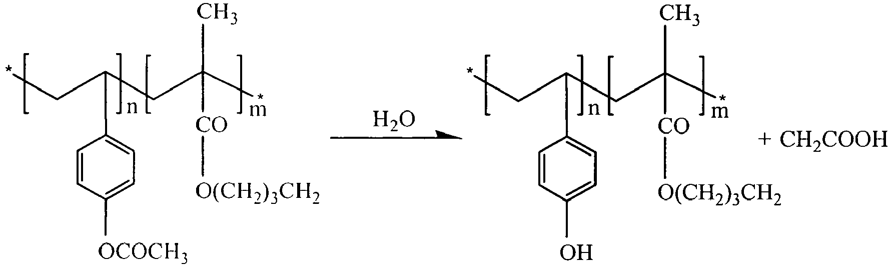

For still another example, various polymerizable monomers with protected BHT functional moieties having the following illustrative formula: 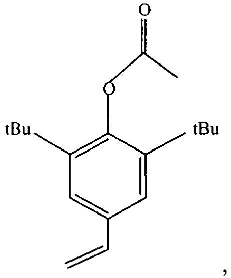 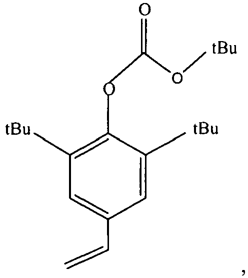 and 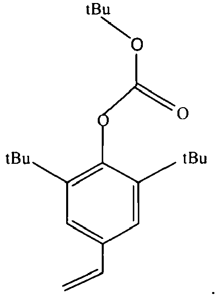 can be polymerized and then hydrolyzed to form a polymer with a covalently bonded active BHT functional moiety, as follows:

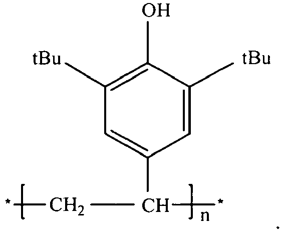

For yet another example, resveratrol, which is a natural phenolic antioxidant typically found in grapes, mulberries, peanuts, and other plants or food products, can be reacted first with tert-butyldimethylchlorosilane (TBDMSCl) in a solvent system comprising imidazole and dry dimethylformamide (DMF) to form a monomeric resveratrol derivative that are protected by -OTBDMS caps, followed by polymerization of the protected resveratrol derivative and hydrolysis in the presence of alkaline and NH₄F, so as to form resveratrol-derived polymers with active phenolic antioxidant functional groups, as follows:

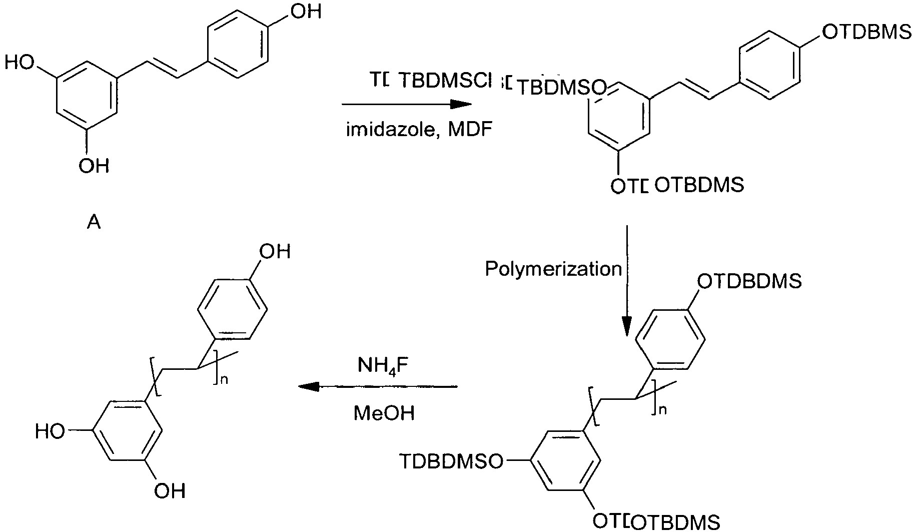

Although trans-resveratrol is primarily illustrated in the above-described reactions, it is readily understood that cis-resveratrol or derivatives of trans- and cis-resveratrol, such as, for example, trans-resveratrol-3-O-glucoside and cis-resveratrol-3-O-glucoside, can also undergo the same reaction with TBDMSCl to form monomeric resveratrol derivatives that are protected by -OTBDMS caps, which then undergo polymerization and hydrolysis to form resveratrol-derived polymers with active phenolic antioxidant functional groups. Exemplary monomeric resveratrol derivatives protected by -OTBDMS caps that can be formed by cis-resveratrol, trans-resveratrol-3-O-glucoside and cis-resveratrol-3-O-glucoside may have the following formula:

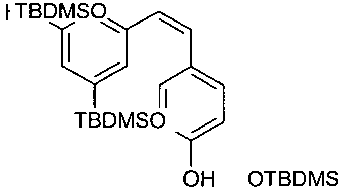

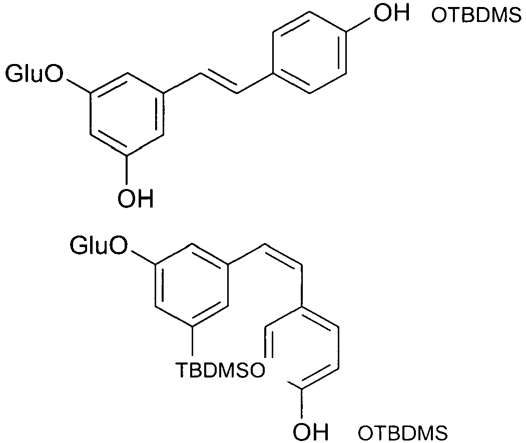

Various polymerization reactions can be used for forming the polymers of the present invention, including but not limited to: free radical reactions, polycondensation reactions, anionic reactions, cationic reactions, etc. Although the above provided illustrative examples primarily show use of free radical reactions for forming the polymers of the present invention, it is understood that the present invention is not so limited and other polymerization techniques can be readily used by a person ordinarily skilled in the art to practice the present invention.

The polymers of the present invention as described hereinabove can be readily mixed with one or more oxidative-susceptible pharmacologically active agents as described hereinabove to form a polymeric composition that contains the pharmacologically active agents as incorporated in a biocompatible polymeric matrix with active antioxidant functional moieties covalently bonded thereto. Further, the biocompatible polymeric matrix may contain one or more conventional polymers, such as poly-BMA, poly-HEMA, BMA/HEMA copolymers, EVAc, etc., that are essentially free of covalently bound active antioxidant functional moieties. The conventional polymers can be readily mixed with the above-described first polymeric material to form polymer blends of desired physical and chemical properties for forming or coating at least a portion of an implantable medical device.

Suitable implantable medical devices that can be formed or coated by the polymers or polymer blends of the present invention include, but are not limited to: stents, stent grafts, anastomosis devices, vascular grafts, vascular patches, shunts, catheters, guide wires, balloons, and filters, which can be used for local administration and/or controlled release of the pharmacologically active agents. Preferably, the present invention provides a stent having at least one section that comprises a polymeric coating formed of one or more polymers or polymer blends of the present invention, for treatment of restenosis and related complications following percutaneous transluminal cornary angioplasty.

## Claims

1. A medical device comprising an implantable structure, wherein at least a portion of said implantable structure comprises or is coated with a polymeric composition comprising a biocompatible polymeric matrix that contains at least one pharmacologically active agent that is susceptible to oxidative degradation, wherein said biocompatible polymeric matrix comprises at least a first polymeric material with an active antioxidant functional moiety covalently bound thereto in sufficient amount for protecting the at least one pharmacologically active agent against oxidative degradation, in which the at least one active antioxidant functional moiety includes trans-resveratrol or cis-resveratrol.

2. The medical device of claim 1, wherein said biocompatible polymeric matrix includes a second polymeric material that is essentially free of covalently bound active antioxidant functional moiety.

3. The medical device of claim 1, wherein the at least one pharmacologically active agent is selected from the group consisting of trans-retinoic acid, paclitaxel, paclitaxel derivatives, rapamycin, and rapamycin derivatives that comprise at least one domain that binds to a mammalian target of rapamycin (mTOR).

4. The medical device of claim 1, wherein said at least one active antioxidant functional moiety is covalently bound to the first polymeric material through a side-chain linking group selected from the group consisting of straight or branched C₁-C₂₀ alkyls, C₁-C₂₀ alkylenes, C₁-C₂₀ oxaalkylenes, C₁-C₂₀ oligo-oxaalkylenes, C₁-C₂₀ alkyleneoxaalkylenes, and C₁-C₂₀ alkylene oligo-oxaalkylenes, optionally with one or more fluorine substitution groups.

5. The medical device of claim 1, wherein the first polymeric material is selected from the group consisting of biocompatible addition polymers and biocompatible condensation polymers.

6. The medical device of claim 4, wherein the first polymeric material is a biostable polymer or a bioabsorbable polymer.

7. The medical device of claim 1, wherein the first polymeric material is selected from the group consisting of biocompatible polyvinyl, polyester, polyamide, poly(esteramide), polyurethane, poly(butylmethacrylate), poly(2-hydroxyethylmethacrylate), or combinations thereof.

8. The medical device of claim 1, wherein the first polymeric material comprises biocompatible polyvinyl.

9. The medical device of claim 1, wherein the first polymeric material comprises a poly(butyl methacrylate) and poly(2-hydroxyethylmethacrylate) copolymer having said at least one active antioxidant functional moiety covalently bounded thereto.

10. The medical device of claim 1, wherein the biocompatible polymeric matrix further comprises the second polymeric material, and wherein the second polymeric material is selected from the group consisting of biocompatible addition polymers and biocompatible condensation polymers.

11. The medical device of claim 10, wherein the second polymeric material is a biostable polymer or a bioabsorbable polymer.

12. The medical device of claim 1, wherein the implantable structure is selected from the group consisting of stents, stent grafts, anastomosis devices, vascular grafts, vascular patches, AV shunts, catheters, guide wires, balloons, and filters.

## Patentansprüche

1. Medizinische Vorrichtung, beinhaltend eine implantierbare Struktur, wobei mindestens ein Abschnitt der implantierbaren Struktur eine polymere Zusammensetzung beinhaltet bzw. damit beschichtet ist, welche eine biokompatible Polymermatrix beinhaltet, welche mindestens einen pharmakologisch wirksamen Stoff beinhaltet, der für oxidativen Abbau anfällig ist, wobei die biokompatible Polymermatrix mindestens ein erstes Polymermaterial mit einem wirksamen funktionalen Antioxidansanteil, welches kovalent hiermit verbunden ist, in hinreichender Menge, um den mindestens einen pharmakologisch wirksamen Stoff vor oxidativem Abbau zu schützen, wobei der mindestens eine wirksame funktionale Antioxidansanteil Trans-Resveratrol oder Cis-Resveratrol enthält.

2. Medizinische Vorrichtung nach Anspruch 1, bei welcher die biokompatible Polymermatrix ein zweites Polymermaterial beinhaltet, das im Wesentlichen frei von kovalent gebundenem, wirksamem Antioxidans-Funktionsanteil ist.

3. Medizinische Vorrichtung nach Anspruch 1, bei welcher der mindestens eine pharmakologisch wirksame Stoff aus der Gruppe, bestehend aus Transretinolsäure, Paclitaxel, Paclitaxel-Derivaten, Rapamycin und Rapamycin-Derivaten, gewählt wird, welcher mindestens eine Domäne beinhaltet, die an ein Ziel des Rapamycins im Säugetier (mTOR) bindet.

4. Medizinische Vorrichtung nach Anspruch 1, bei welcher der mindestens eine wirksame Antioxidans-Funktionsanteil kovalent an das erste Polymermaterial durch eine Seitenkettenverbindungsgruppe gebunden ist, welches aus der Gruppe, bestehend aus linearen oder verzweigten C₁-C₂₀ Alkylen, C₁-C₂₀ Alkylenen, C₁-C₂₀ Oxaalkylenen, C_{I}-C₂₀ Oligo-Oxaalkylenen, C₁-C₂₀ Alkylenoxaalkylenen und C₁-C₂₀ Alkylen-Oligo-Oxaalkylenen, optionsweise mit einer oder mehreren Fluorsubstitutionsgruppen, gewählt wird.

5. Medizinische Vorrichtung nach Anspruch 1, bei welcher das erste Polymermaterial aus der Gruppe, bestehend aus biokompatiblen Additionspolymeren und biokompatiblen Kondensationspolymeren, gewählt wird.

6. Medizinische Vorrichtung nach Anspruch 4, bei welcher das erste Polymermaterial ein biostabiles Polymer oder ein bioabsorbierbares Polymer ist.

7. Medizinische Vorrichtung nach Anspruch 1, bei welcher das erste Polymermaterial aus der Gruppe, bestehend aus biokompatiblem Polyvinyl, Polyester, Polyamid, Poly(esteramid), Polyurethan, Poly(butylmethacrylat), Poly(2-hydroxyethylmethacrylat), oder Kombinationen hieraus, gewählt wird.

8. Medizinische Vorrichtung nach Anspruch 1, bei welcher das erste Polymermaterial biokompatibles Polyvinyl beinhaltet.

9. Medizinische Vorrichtung nach Anspruch 1, bei welcher das erste Polymermaterial ein Poly(butylmethacrylat)- und Poly(2-hydroxyethylmethacrylat)-Copolymer beinhaltet, welches den mindestens einen kovalent hiermit verbundenen wirksamen Antioxidans-Funktionsanteil besitzt.

10. Medizinische Vorrichtung nach Anspruch 1, bei welcher die biokompatible Polymermatrix zudem das zweite Polymermaterial beinhaltet, und bei welcher das zweite Polymermaterial aus der Gruppe, bestehend aus biokompatiblen Additionspolymeren und biokompatiblen Kondensationspolymeren, gewählt wird.

11. Medizinische Vorrichtung nach Anspruch 10, bei welcher das zweite Polymermaterial ein biostabiles Polymer oder ein bioabsorbierbares Polymer ist.

12. Medizinische Vorrichtung nach Anspruch 1, bei welcher die implantierbare Struktur aus der Gruppe, bestehend aus Stents, Stent-Implantaten, Anastomose-Vorrichtungen, Gefäßimplantaten, Gefäß-Patchs, AV-Shunts, Kathetern, Führungsdrähten, Ballons und Filtern, gewählt wird.

## Revendications

1. Dispositif médical comprenant une structure implantable, dans lequel au moins une partie de ladite structure implantable comprend ou est revêtue par une composition polymérique qui comprend une matrice polymérique biocompatible qui contient au moins un agent pharmacologiquement actif qui est susceptible d'une dégradation oxydative, dans lequel ladite matrice polymérique biocompatible comprend au moins un premier matériau polymérique avec une moitié fonctionnelle anti-oxydante active qui lui est liée de manière covalente selon une quantité suffisante pour protéger l'au moins un agent pharmacologiquement actif contre une dégradation oxydative, dans lequel l'au moins une moitié fonctionnelle anti-oxydante active inclut du trans-resvératrol ou du cis-resvératrol.

2. Dispositif médical selon la revendication 1, dans lequel ladite matrice polymérique biocompatible inclut un second matériau polymérique qui est essentiellement exempt de moitié fonctionnelle anti-oxydante active liée de manière covalente.

3. Dispositif médical selon la revendication 1, dans lequel l'au moins un agent pharmacologiquement actif est sélectionné parmi le groupe constitué par l'acide transrétinoïque, le paclitaxel, les dérivés de paclitaxel, la rapamycine et les dérivés de rapamycine qui comprennent au moins un domaine qui se lie à une cible de la rapamycine chez les mammifères (mTOR).

4. Dispositif médical selon la revendication 1, dans lequel ladite au moins une moitié fonctionnelle anti-oxydante active est liée de manière covalente au premier matériau polymérique par l'intermédiaire d'un groupe de liaison en chaîne latérale sélectionné parmi le groupe constitué par les C₁-C₂₀ alkyles, les C₁-C₂₀ alkylènes, les C₁-C₂₀ oxaalkylènes, les C₁-C₂₀ oligo-oxaalkylènes, les C₁-C₂₀ alkylèneoxaalkylènes et les C₁-C₂₀ alkylène oligo-oxaalkylènes en chaîne droite ou ramifiés, en option avec un ou plusieurs groupe(s) de substitution fluor.

5. Dispositif médical selon la revendication 1, dans lequel le premier matériau polymérique est sélectionné parmi le groupe constitué par les polymères d'addition biocompatibles et les polymères de condensation biocompatibles.

6. Dispositif médical selon la revendication 4, dans lequel le premier matériau polymérique est un polymère bio-stable ou un polymère bio-absorbable.

7. Dispositif médical selon la revendication 1, dans lequel le premier matériau polymérique est sélectionné parmi le groupe constitué par le polyvinyle, le polyester, le polyamide, le poly(esteramide), le polyuréthane, le poly(butylméthacrylate), le poly(2-hydroxyéthylméthacrylate) biocompatibles ou des combinaisons de ceux-ci.

8. Dispositif médical selon la revendication 1, dans lequel le premier matériau polymérique comprend du polyvinyle biocompatible.

9. Dispositif médical selon la revendication 1, dans lequel le premier matériau polymérique comprend un copolymère poly(butylméthacrylate) et poly(2-hydroxyéthylméthacrylate) qui comporte ladite au moins une moitié fonctionnelle anti-oxydante active qui lui est liée de manière covalente.

10. Dispositif médical selon la revendication 1, dans lequel la matrice polymérique biocompatible comprend en outre le second matériau polymérique, et dans lequel le second matériau polymérique est sélectionné parmi le groupe constitué par les polymères d'addition biocompatibles et les polymères de condensation biocompatibles.

11. Dispositif médical selon la revendication 10, dans lequel le second matériau polymérique est un polymère bio-stable ou un polymère bio-absorbable.

12. Dispositif médical selon la revendication 1, dans lequel la structure implantable est sélectionnée parmi le groupe constitué par les endoprothèses, les implants endoprothétiques, les dispositifs pour anastomose, les implants vasculaires, les pièces vasculaires, les shunts AV, les cathéters, les fils de guidage, les ballons et les filtres.
